# EUROPEAN PATENT SPECIFICATION

(11) **EP 2 994 536 B1**
(45) Date of publication and mention of the grant of the patent: **14.06.2017**
(21) Application number: 14722216.0
(22) Date of filing: 08.05.2014
(51) Int. Cl.: C12Q 1/00, C12Q 1/32, C12N 9/96, G01N 33/52

(54) **STABILIZATION OF ENZYMES BY NICOTINIC ACID**
STABILISIERUNG VON ENZYMEN DURCH NIKOTINSÄURE
STABILISATION D'ENZYMES PAR DE L'ACIDE NICOTINIQUE

(30) Priority: 08.05.2013 EP 13167046
(43) Date of publication of application: 16.03.2016
(73) Proprietor: Roche Diabetes Care GmbH, 68305 Mannheim (DE); F. Hoffmann-La Roche AG, 4070 Basel (CH)
(72) Inventor: CHEMNITIUS, Gabriele, 68199 Mannheim (DE); NAGEL, Thomas, 68309 Mannheim (DE); GAA, Otto, 67551 Worms (DE); RECHT, Karl, 68642 Bürstadt (DE); MEIER, Thomas, 81373 München (DE)
(74) Representative: Herzog, Fiesser & Partner Patentanwälte PartG mbB
(86) International application number: PCT/EP2014/059418
(87) International publication number: WO 2014/180939

(56) References cited:
- EP-A1- 2 017 608
- WO-A2-2007/072013
- US-A1- 2011 143 416
- HACHISUKA Y ET AL: "The action of dipicolinic acid and its chemical analogues on the heat stability of glucose dehydrogenase of Bacillus subtilis spores.", JOURNAL OF BIOCHEMISTRY MAY 1967, vol. 61, no. 5, May 1967 (1967-05), pages 659-661, XP009173082, ISSN: 0021-924X cited in the application

## Description

The present invention is concerned with means and methods for maintaining and preserving enzymatic activity. In particular, the invention relates to a dry composition comprising a dehydrogenase, a redox cofactor, an agent capable of eliciting at least one measurable change in a property of an indicator reagent in the presence of redox equivalents, an indicator reagent, and at least one pyridine-carboxylic acid or a derivative or salt thereof. The invention further contemplates a diagnostic test element for the determination of an analyte from a body fluid sample and a method for the manufacture of such a test element. Further envisaged by the present invention is the use of a pyridine-carboxylic acid or a derivative thereof for reducing a decrease of the enzymatic activity of at least one enzyme in a composition under dry and/or under humid conditions. Furthermore, contemplated is a method for determining the presence or amount analyte in a body fluid sample based on the test element according to the invention.

Diagnostic test elements are usually manufactured for use in near-patient applications. Therefore, the elements must be robust with respect to handling and storage. This applies, in particular, for the test chemistry of the test elements. (see Hones 2008, Diabetes Technology & Therapeutics 10: S10)

However, many diagnostic test elements are based on a rather complex enzyme test chemistry present on the test element. In particular, the test element comprises a carrier and a detection layer wherein the detection layer usually contains enzymes. It is decisive for the proper function of the test elements that these enzymes remain biologically active during storage and upon treatment. Since calibration for an individual measurement is usually not possible, the test elements are normally calibrated batch-wise. The calibration information for a batch of test elements is stored and used for each element of the batch regardless of individual differences in treatment and storage.

However, pretreatments of the test elements and storage conditions can severely affect the activity of the enzymes. For example, heat treatment, either during the manufacture or the storage of the test elements, can denature the enzymes such that the overall enzymatic activity present on a test element is significantly reduced which, in turn, will result in wrong test results when such a test element is used. Similarly, many enzymes are sensitive with respect to oxidation processes which also results in denaturation and irreversible enzyme inactivation. However, many enzymes on test elements are present, at least during the time of storage, in a solvent-free environment which may promote even such oxidation processes. Moreover, the detection layer may comprise additional components which even more facilitate oxidation processes such as redox cofactors and other redox relevant components and the like.

The problem of preserving enzymatic activities under such unfavorable conditions, however, does not only apply for test elements. Rather, more generally, many enzyme preparations are provided and stored in essentially solvent-free form, such as freeze-dried preparations.

Various so-called compatible solutes, i.e., low molecular compounds of different chemical classes, such as sugars, polyols, free amino acids, amino acid derivatives, amines and sulphur analoga, sulfate esters, short peptides and cyclic 2,3-diphosphoglycerate, have been investigated for their preservative properties for proteins in solution and under dry conditions (see Arakawa 1985, Biophys J 47: 411; Lippert 1992, Appl Microbiol Biotechnol 37: 61; Göller 1999, J Mol Catal B Enzymatic 7:37; Lentzen 2006, Appl Microbiol Biotechnol 72: 623; WO2007/002657, US2010/0255120).

Stabilization of glucose oxidase by compatible solutes like ectoine and hydroxyectoine in biosensors for the electrochemical determination of glucose in solution has been previously reported (WO2007/097653; Loose 2006, Proceedings of the 24th IASTED International Multi-conference Biomedical Engineering, 167-173, Innsbruck, AT). However, glucose oxidase is known to be a rather stable enzyme with respect to oxidative stress and heat. On the other hand, pyridine-carboxylic acids and derivatives thereof have been reported to be insufficient to effectively prevent inactivation of, e.g., glucose dehydrogenase in solution (Hachisuka et al. (1967), J Biochem 61: 659).). Dehydrogenases in general and, in particular, glucose dehydrogenases are rather sensitive enzymes for oxidative stress and heat treatment. However, they are important diagnostic tools.

Thus, there is a need for preservation of the enzymatic activity of storage and temperature sensitive enzymes used for diagnostic applications and, in particular, for the class of dehydrogenases, such as glucose dehydrogenase.
The technical problem underlying the present invention could be regarded as the provision of means and methods for complying with the aforementioned needs. The problem is solved by the embodiments defined in the claims.
Thus, the present invention relates to a dry composition comprising the components:
(a) a dehydrogenase;
(b) a redox cofactor;
(c) an agent capable of eliciting a change in at least one measurable property of an indicator reagent in the presence of redox equivalents;
(d) an indicator reagent; and
(e) at least one pyridine-carboxylic acid or a salt thereof, wherein said pyridine-carboxylic acid is a pyridine-monocarboxylic acid or a pyridine-dicarboxylic acid. The term "composition", as used herein, relates to a mixture comprising the compounds as specified herein. It is understood by the skilled person that the composition may comprise additional components, e.g., preferably, buffer components (e.g., of phosphate buffered saline, Tris buffer, citrate buffer, glycerine phosphate buffer, or Good's buffer) or other salts, detergents, or the like, including the components as specified herein below.

The term "dry composition" as used herein means that the composition is essentially free of a solvent or a mixture of solvents. Essentially free as used herein means that at least 85%, at least 90%, at least 92%, at least 95%, or at least 98% of the solvent or solvent mixture which was originally present in a solution of the composition has been removed from the composition. Accordingly, it is, preferably, envisaged that the solvent or solvent mixture is present in the dry composition of the invention in an amount of up to 15%, and preferably, up to 10%, up to 8%, up to 5%, or up to 2%. More preferably, a dry composition is a composition comprising water in an amount of up to 15%, and most preferably, up to 10%, up to 8%, up to 5%, or up to 2%. Methods for determining residual water are known to the skilled person; preferably, residual water in a composition according to the present invention is determined using a phosphorus pentoxid sensor according to the method as described in WO 1993/012418. The aforementioned percentage values and the other percentage values referred herein used in order to define amounts refer to percent by weight (w/w). Such a composition of the invention is, preferably, a solid composition under normal conditions, i.e., under room temperature and normal pressure.

A solvent in accordance with the present invention is a solvent which allows for dissolving the components of the composition of the present invention under conditions which do not irreversibly impair the function of the components of the composition and, in particular, the enzymatic activity of the dehydrogenase. Moreover, it is envisaged that the solvent dissolves the components, preferably, under standard pressure, preferably 1 bar +/- 10% within a temperature range of 5 to 50°C and, more preferably, room temperature, preferably 20°C +/-10°C. Suitable solvents according to the present invention include, preferably, water and alcohols, such as hexanol, 2-methoxy-propanol, 2-methyl-2-butanol. It will be understood that in accordance with the present invention, a solvent to be used can be a mixture of two or more of the aforementioned solvents. Preferred solvent mixtures envisaged in this context are mixtures of water or water-based buffers with alcohols and in particular, the solvent mixtures referred to in the accompanying Examples, below.

A dry composition according to the present invention can be provided, preferably, by dissolving the components of the composition of the present invention first in a solvent or mixture of solvents and subsequently removing the said solvent or mixture of solvents by a suitable treatment such that the remaining composition is essentially free of the said solvent or solvent mixture. Suitable treatments to be preferably envisaged by the present invention include heat treatment, evaporation techniques, freeze drying and the like. Preferably, the envisaged treatment is heat treatment and, in particular, heat treatment under the following conditions: heat treatment at about 60°C or more for approximately 20 to 45 minutes or at about 95°C for approximately 1 to 2 minutes with heat circulation; thickness of the composition of 20 to 200 micrometers or less; at a pressure of 1 bar or 0.1 bar. Moreover, it will be understood that in order to keep the composition under dry conditions, storage is, preferably, carried out in the presence of a drying agent, i.e., a desiccant. Suitable drying agents, preferably, encompass silica gel, zeolites, calcium carbonate or magnesium sulfate.

The term "dehydrogenase" as used herein refers to a polypeptide which is capable of catalyzing the oxidation or reduction of a substrate by transferring hydrides (H⁻) as redox equivalents to or from a redox cofactor as referred to herein elsewhere. Preferably, a dehydrogenase is a polypeptide which is capable of catalyzing the oxidation of a substrate by transferring hydrides (H-) as redox equivalents to an acceptor molecule, preferably, to a redox cofactor as referred to herein elsewhere. Dehydrogenases envisaged by the present invention are, preferably, those which depend on a redox cofactor (or sometimes referred to as co-enzyme) such as pyrrolo quinoline quinone (PQQ), nicotinamide-adenine-dinucleotide (NAD) or a derivative thereof, or a flavine cofactor, such as flavin-adenine-dinucleotide (FAD) or flavine mononucleotide (FMN). Preferred dehydrogenases are, in particular, lactate dehydrogenase (EC number 1.1.1.27 or 1.1.1.28), glucose dehydrogenases (see below), alcohol dehydrogenase (EC number EC number 1.1.1.1 or 1.1.1.2), L-amino acid dehydrogenase (EC number 1.4.1.5), glycerin dehydrogenase (EC number 1.1.1.6), malate dehydrogenase (EC number 1.1.1.37), 3-hydroxybutyrate dehydrogenase (EC number 1.1.1.30), or sorbitol dehydrogenase (EC number 1.1.1.14).

More preferably, said dehydrogenase is a glucose dehydrogenase. Most preferably, said glucose dehydrogenase is selected from the group consisting of: glucose dehydrogenase (EC number 1.1.1.47), quinoprotein glucose dehydrogenase (EC number 1.1.5.2), in particular, Pyrrolo quinoline quinone (PQQ)-dependent glucose dehydrogenase (EC number 1.1.5.2), glucose-6-phospate dehydrogenase (EC number 1.1.1.49), nicotinamide adenine dinucleotide (NAD)-dependent glucose dehydrogenase (EC number 1.1.1.119) and flavin adenine dinucleotide (FAD)-dependent glucose dehydrogenase (EC number 1.1.99.10) or enzymatically active mutants thereof.

Enzymatically active mutants of the aforementioned enzymes can be obtained by substituting, adding or deleting one or more amino acids from the amino acid sequences reported for the aforementioned wild type enzymes in the prior art as recited before. Preferred mutants are the mutants of the PQQ-dependent glucose dehydrogenase having an improved substrate specificity compared to their wild type counterparts as disclosed in US 7,132,270 or US 7,547,535. Both documents are herewith incorporated by reference with respect to the mutants. Further mutants are those disclosed in Baik et al (Baik 2005, Appl Environ Microbiol 71: 3285), Vasquez-Figuera et al. (Vasquez-Figuera 2007, Chem BioChem 8: 2295), and WO 2005/045016.

Preferred in accordance with the present invention is a glucose dehydrogenase (E.C. 1.1.1.47) mutant disclosed in WO2009/103540A1, p.21) having a mutation at least at amino acid positions 96, 170 and/or 252, herewith incorporated by reference. Preferred mutations envisaged at theses amino acid positions are substitutions of Glu96Gly, Glu170Arg or Lys and/or Lys252Leu.

The term "redox cofactor" as used herein refers to a molecule which can serve as an acceptor for enzymatically transferred redox equivalents and, in particular, hydride (H-). Preferably, the redox cofactor is PQQ, NAD or FAD. It will be understood that the redox cofactor to be included in the composition of the present invention depends on the properties of the dehydrogenase to be envisaged. For example, PQQ is combined in a composition according to the present invention with a PQQ dependent glucose dehydrogenase, NAD is combined in a composition according to the present invention with a NAD dependent glucose dehydrogenase, and FAD is combined in a composition according to the present invention with a FAD dependent glucose dehydrogenase. A redox cofactor according to the present invention may also preferably be a derivative of PQQ, NAD or FAD. Preferred derivatives of NAD are those disclosed in WO 2007/012494 which is herewith incorporated by reference with respect to the disclosed NAD/NADH and/or NADP/NADPH derivatives. More preferably, the redox cofactor in accordance with the present invention is carbaNAD as disclosed in WO 2007/012494.

The term "redox equivalents" as used herein relates to the concept commonly used in redox chemistry well known to the skilled person. Preferably, the term relates to electrons which are transferred from a substrate of the dehydrogenase to the redox cofactor or electrons transferred to the indicator reagent from the redox cofactor.

The term "measurable property", as used herein, relates to any property of the composition which changes in the presence of the analyte and which can be transferred into a physical signal of any kind. Preferably, the change of the measurable property and/or the signal which can be generated therefrom are proportional to the concentration of the analyte in the sample.

Preferably, the measurable property is an electrochemical property. Thus, it is envisaged by the present invention that the composition includes one or more chemical reagents for reacting with the analyte to produce an electrochemical signal that represents the presence of the analyte in the sample fluid. Preferably, electrochemical properties include amperometric or coulometric responses indicative of the concentration of the analyte. See, for example, U.S. Pat. Nos. 5,108,564, 4,919,770 and 6,054,039.

More preferably, the measurable property is an optical property, i.e., a property which can be detected by an optical instrument. Preferably, an optical property as referred to herein refers to a property of the indicator reagent which can be optically detected such as light absorption or emission, remission, refraction or polarization and properties associated therewith. It will be understood that such a change of at least one optical property as used herein encompasses the detection of the presence of a property which was not detectable before, the detection of the absence of a property which has been detected before, and the detection of quantitative changes of a property, i.e., the detection of the change of the signal strength which correlates to the extent of the change of the at least one optical property. Preferred optical properties envisaged by the present invention are color, fluorescence, luminescence, or refractometry. The optical properties which are to be changed by the agent envisaged according to the present invention depend on the type of indicator reagent. Dependent on the desired optical property to be detected and the agent to be used in the composition, the skilled person is in a position to select without further ado a suitable indicator reagent, in particular among those referred to herein elsewhere. Methods of converting the optical property as defined above into a physical signal which can be read as a measurement value are well known in the art and are described, e.g., in EP 0 821 234, EP 0 974 303, and US 2005/0023152.

Accordingly, the term "agent capable of eliciting a change in at least one measurable property of an indicator reagent in the presence of redox equivalents" refers to a molecule which, in the presence of the redox equivalents, is capable of inducing a change in at least one measurable property in an indicator reagent. It is to be understood in accordance with the present invention that the agent may also elicit a change in more than one measurable property of the indicator reagent which may then subsequently be detected. Furthermore, the agent may, preferably, also elicit a change in measurable properties of more than one indicator reagent which may then subsequently be detected.

An agent as referred to above is, preferably, capable of transferring directly or indirectly, i.e., via a further mediator, redox equivalents from the redox cofactor to the indicator reagent. As a consequence of the said transfer of the redox equivalents, the indicator reagent will be modified such that a change in at least one measurable property occurs. In case the measurable property is an optical property, preferably, for example, a color-less or non-fluorescing indicator reagent in an oxidized state may be converted into a colored or fluorescent indicator reagent by the transfer of redox equivalents mediated by the agent in a reduced state. The transfer of the redox equivalents may be direct in that the redox equivalents are transferred by the agent to the indicator reagent or may be indirect. In the latter case, the redox equivalents are transferred from the agent to an intermediate mediator which subsequently transfers the redox equivalents to the indicator reagent. It will be understood that, preferably, more than one mediator can be used. For example, the agent may transfer the redox equivalents to a first mediator which subsequently transfers the redox equivalents to a second mediator and said second mediator than transfers the redox equivalents to the indicator reagent. It will be understood that in such a mediator cascade more than two mediators could be used. An advantage of using one or more mediators for the transfer of the redox equivalents to the indicator reagent is that the timing of the detection of the measurable property can be improved.

Mediators which can be applied in the context of the present invention are well known in the art and include, e.g., potassium ferricyanide, quinone derivatives, Nile blue (CAS no.: 3625-57-8), Meldola's blue (CAS no.: 7057-57-0), osmium complexes as disclosed in EP 1 457 572 B1, or transition metal complexes such as ruthenium hexamine chloride.

An agent according to the invention which is, preferably, envisaged is a phenazine. More preferably, said phenazine is phenazinethosulfate, phenazinmethosulfate, 1-(3-carboxypropoxy)-5-ethylphenaziniumtrifluoromethansulfonate or 1-methoxyphenazine-methosulfate. Such phenazines can be applied for eliciting a change in at least one optical property of an indicator reagent. Details for the detection and on how such phenazines are to be applied can be found in EP 0 654 079 A1. Also, an agent envisaged in this context is, preferably, a chinone. More preferably, the said chinone is phenanthrenchinone, phenanthrolinchinone or benzo[h]-chinolinchinone. Another agent envisaged in this context is a nitrosoaniline. More preferably, said nitrosoaniline is [(4-nitrosophenyl)imino]dimethanol-hydrochloride. Also preferably envisaged by the present invention is an agent capable of eliciting a change in at least one measurable property of an indicator reagent in the presence of redox equivalents, said agent being an enzyme capable of catalyzing the transfer of redox equivalents from the redox cofactor to the indicator reagent.

The term "indicator reagent" as used herein refers to a molecule or molecular entity which as a consequence of the transfer of redox equivalents will be modified such that a change in at least one measurable property occurs.
Indicator reagents which, as a consequence of the transfer of redox equivalents, will be modified such that a change in at least one electrochemical property occurs are known in the art (e.g. Methods of Enzymatic Analysis; (ed. H.G: Bergmeyer), Chapter 2.6.; Vol. I, p.197ff.; Weinheim 1983). Preferred indicator reagents to be applied in the composition of the invention encompass heteropoly acids, preferably, 2,18-phosphoromolybdenic acid, chinones, preferably resazurine, dichlorophenolindophenole, and/or tetrazolinum salts, preferably, the commercially available WST-3, WST-4 and WST-5 salts (Dojindo, Inc. US). These indicator reagents are reduced upon transfer of the redox equivalents and this reduction is accompanied by a change in at least one optical property and, in particular, the color.
Further preferred indicator reagents envisaged in accordance with the present invention are reagents, such as fluorophores, the fluorescence of which is changed upon transfer of redox equivalents. Suitable fluorophores include, among others, the flavine nucleotides and nicotin-adenine-dinucleotides referred to herein also in the context of the redox cofactors. If a redox cofactor such as carbaNAD or NAD is applied in the composition of the invention as an indicator reagent, the components (b), (c) and (d) of the composition of the invention may all be represented by the same molecule, i.e., the carbaNAD or NAD. Accordingly, the components (b), (c) and (d) may be represented in the composition of the invention by the same chemical entity. Moreover, a modified nitrosoaniline as disclosed in EP 0 620 283 B1 or EP 0 831 327 B1, the respective disclosure content of which is herewith incorporated by reference, can be used, preferably, as component (c) and component (d). Thus, components (c) and (d) may be represented in the composition of the invention by the same chemical entity.
The term "pyridine-carboxylic acid" is known to the skilled person and, according to the present invention, includes pyridine-mono- and dicarboxylic acids and salts thereof. Preferably, the term relates to a pyridine-dicarboxylic acid, e.g., 2,4-Pyridinedicarboxylic acid (lutidinic acid); 2,5-Pyridinedicarboxylic acid (isocinchomeronic acid); 2,6-Pyridinedicarboxylic acid (dipicolinic acid); 2,3-Pyridinedicarboxylic acid (quinolinic acid), 3,4-Pyridinedicarboxylic acid; or 3,5-Pyridinedicarboxylic acid. More preferably, the term relates a pyridine-monocarboxylic acid. Most preferably, the term relates to pyridine-3-carboxylic acid (nicotinic acid, niacin, vitamin B3, CAS number 59-67-6), pyridine-4-carboxylic acid (isonicotinic acid, CAS number 55-22-1), pyridine-2-carboxylic acid (picolinic acid, CAS number 98-98-6), or the sodium and potassium salts thereof. The pyridine-carboxylic acid has the activity of preventing a reduction of enzymatic activity of at least one dehydrogenase enzyme as detailed herein below.

Preferably, the pyridine-carboxylic acid reduces a decrease of the enzymatic activity of at least one dehydrogenase enzyme in the composition under dry or humid conditions. The at least one enzyme can be, however, also both, the dehydrogenase as well as an enzyme which is applied in the composition of the present invention as an agent capable of eliciting a change in at least one measurable property of an indicator reagent in the presence of redox equivalents. More preferably, the decrease of the enzymatic activity is prevented or at least significantly reduced in the composition of the invention during manufacture and/or storage at room temperature or even higher temperatures as specified below by the at least one pyridine-carboxylic acid or derivative thereof in comparison to a control composition without said at least one pyridine-carboxylic acid or derivative thereof, such that at least 40%, at least 50%, at least 60%, at least 70%, at least 80% or at least 90% of the enzymatic activity of one or both enzymes is maintained. Preferably, said residual activity is still present after two, three, four, five, six, seven, eight, nine, ten, eleven, twelve, 13, 14, 15, 20, or 25 weeks. Thus, e.g., preferably, at least 60% of the enzymatic activity of one or both enzymes is maintained after two, three, four, five, six, seven, eight, nine, ten, eleven, twelve, 13, 14, 15, 20, or 25 weeks; or at least 70% of the enzymatic activity of one or both enzymes is maintained after two, three, four, five, six, seven, eight, nine, ten, eleven, twelve, 13, 14, 15, 20, or 25 weeks; or at least 80% of the enzymatic activity of one or both enzymes is maintained after two, three, four, five, six, seven, eight, nine, ten, eleven, twelve, 13, 14, 15, 20, or 25 weeks; or at least 90% of the enzymatic activity of one or both enzymes is maintained after two, three, four, five, six, seven, eight, nine, ten, eleven, twelve, 13, 14, 15, 20, or 25 weeks.
The skilled person understands that the residual activity of an enzyme stored for an extended time period depends on several factors, as described herein. E.g., the residual activity may depend on duration of storage, temperature, humidity, oxidative stress, irradiation, and the like. Thus, preferably, the pyridine-carboxylic acid reduces a decrease of the enzymatic activity of at least one enzyme in the composition under dry conditions. As used herein, the term "dry conditions" relates to conditions wherein the composition of the present invention is stored under and, preferably, is equilibrated with, an atmosphere having a relative humidity of 0% - 50%, 10% - 50%, 20% - 50%, 30% - 50%, 0% - 40%, 0% - 30%, or 0% - 20%. Also preferably, the pyridine-carboxylic acid reduces a decrease of the enzymatic activity of at least one enzyme in the composition under humid conditions. As used herein, the term "humid conditions" relates to conditions wherein the composition of the present invention is stored under and, preferably, is equilibrated with, an atmosphere having a relative humidity of 50% - 100%, 50% - 90%, 50% - 85%, 60% - 100%, 75% - 100%, 75% - 100%, or 85% - 90%. Preferably, the at least one enzyme in the composition according to the present invention maintains the aforesaid residual activity upon storage at more than 0°C, more than 5°C, more than 10°C, more than 20°C, more than 30°C, or more than 40°C for at least one of the time frames and at least one of the humidity ranges as referenced above. Also preferably, the at least one enzyme in the composition according to the present invention maintains the aforesaid residual activity upon storage at at most 30°C, at most 35°C, at most 40°C, at most 45°C, or at most 50°C for at least one of the time frames and at least one of the humidity ranges as referenced above. It is understood by the skilled person that the temperature ranges recited above can be combined to closed value ranges, e.g., preferably, the at least one enzyme in the composition according to the present invention maintains the aforesaid residual activity upon storage at 0°C - 30°C, 0°C - 35°C, 0°C - 40°C, 0°C - 45°C, 0°C- 50°C, 5°C - 30°C, 5°C - 35°C, 5°C - 40°C, 5°C - 45°C, 5°C- 50°C, 10°C - 30°C, 10°C - 35°C, 10°C - 40°C, 10°C - 45°C, 10°C- 50°C, 20°C - 30°C, 20°C - 35°C, 20°C - 40°C, 20°C - 45°C, 20°C- 50°C, 30°C - 35°C, 30°C - 40°C, 30°C - 45°C, 30°C- 50°C, 40° C-45°C, or 40°C- 50°C C for at least one of the time frames and at least one of the humidity ranges as referenced above.

It is understood by the skilled person that in certain embodiments a change in one of the above referenced conditions may have an impact on the effect of a second condition; e.g., an increase in temperature may aggravate the effect of increasing humidity on the stability of the at least one enzyme of the present invention. Thus, preferably, the pyridine-carboxylic acid significantly reduces a decrease of the enzymatic activity of at least one enzyme in the composition stored under and, preferably, equilibrated with, a temperature of 20°C - 70°C and a relative humidity of 60% to 100%; more preferably, the pyridine-carboxylic acid significantly reduces a decrease of the enzymatic activity of at least one enzyme in the composition stored under and, preferably, equilibrated with, a temperature of 25°C - 60°C and a relative humidity of 70% to 100%; even more preferably, the pyridine-carboxylic acid significantly reduces a decrease of the enzymatic activity of at least one enzyme in the composition stored under and, preferably, equilibrated with, a temperature of 30°C - 55°C and a relative humidity of 80% to 95%; most preferably, the pyridine-carboxylic acid significantly reduces a decrease of the enzymatic activity of at least one enzyme in the composition stored under and, preferably, equilibrated with, a temperature of 35°C - 45°C and a relative humidity of 85% to 90%.
In a preferred embodiment of the composition of the present invention, the pyridine-carboxylic acid is present in amounts of at least 0.4 (w/w)%, at least 0.8 (w/w)%, at least 1.7 (w/w)%, at least 3 (w/w)%, at least 4 (w/w)%, at least 5 (w/w)%, at least 6 (w/w)%, at least 7 (w/w)% or at least 8 (w/w)%.
In a preferred embodiment of the composition of the present invention, said composition further comprises at least one detergent, swelling agent, film-forming agent, and/or solid particle. Suitable stabilizers, detergents, swelling agents, film forming agents, oxidizing agents, and/or solid particles to be used in the composition of the invention are known to the skilled artisan.
Preferably, the said at least one detergent is selected from the group consisting of: Sodium-N-methyl-N-oleoyltaurat, N-octanoyl-N-methyl-glucamid, Mega 8 (N-methyl-N-octanoylglucamide), dioctylsodium sulfosuccinate (DONS), Rhodapex® (preferably CO-433 or CO-436).

Preferably, said at least one swelling agent is selected from the group consisting of: methyl vinyl ether maleic acid anhydride copolymer, xanthan gum and methyl vinyl ether maleic acid copolymer.
Preferably, said at least one film-forming agent is selected from the group consisting of: polyvinylpropionate dispersions, Polyvinyl esters, polyvinyl acetates, polyacrylic esters, polymethacrylic acid, polyvinyl amides, polyamides, polystyrene and mixed polymerizates are also suitable such as of butadiene, styrene or maleic acid ester.
Preferably, said at least one solid particle is selected from the group consisting of: silica particles, in particular, silicon dioxide, sodium silicates or aluminium silicates, kieselguhr, metal oxides, in particular, titan oxide and/or aluminium oxide, synthetic oxide materials, in particular, nanoparticles of oxide materials such as nanoparticles of silicon dioxide, aluminium oxide, or titan oxide, Kaolin, powder glass, amorphous silica, calcium sulfate, and barium sulfate.
In a particular preferred embodiment of the composition of the invention, said composition comprises and, even more preferably, essentially consists of the components listed in Table 1 in the accompanying Examples.
The procedures for removing solvent from a composition referred to herein above and, in particular, the heat treatment, are known to affect the enzymatic activity, in particular, the enzymatic activity of sensitive enzymes such as dehydrogenases. Moreover, under dry conditions, enzymes such as dehydrogenases become more sensitive for oxidation processes and accompanying enzyme denaturation (Andersson 2000, Biotechnol. Appl. Biochem 32: 145-153). Accordingly, the manufacture of complex, dry compositions for enzymatic detection assays as well as the storage thereof is often accompanied by increasing inactivation of the enzymes by denaturation, aggregation or other processes. Upon reconstitution of the enzymes in a solvent-containing surrounding, a reduced enzymatic activity is often observed. Advantageously, it has been found in the studies underlying the present invention that the reduction of the said enzymatic activity which occurs during manufacture and/or storage of the complex dry compositions of the present invention can be significantly prevented by the addition of a pyridine-carboxylic acid as specified elsewhere herein in detail. The finding is surprising since pyridine-carboxylic acids and derivatives thereof have been reported to be insufficient to effectively prevent inactivation of, e.g., glucose dehydrogenase in solution (Hachisuka et al. (1967), J Biochem 61: 659). Moreover, it is also surprising that the preservative effect occurs even under the redox sensitive conditions present in the rather complex, solvent-free composition of the present invention. In particular, the enzymatic activity present in a dry composition having the components of the composition of the invention except for the pyridine-carboxylic acid has been found in the studies underlying the present invention to decrease during storage under dry or humid conditions and temperatures above 4°C. However, significantly higher enzymatic activities could be maintained in the studies underlying the present invention when a pyridine-carboxylic acid was present in the composition. A further advantage of the composition of the present invention is that the pyridine-carboxylic acid in the composition does not interfere with a detection system for a measurable property. In particular, it does not interfere with the measurable signals generated and also does not impair the stability or function of the indicator reagent, the redox cofactor or the agent capable of eliciting the at least one measurable change. Moreover, the enzymatic conversion and conversion rates are, preferably, not impaired by the pyridine-carboxylic acid. All definitions and explanations for the terms made herein above apply mutatis mutandis for the embodiments described as follows. Additional definitions and explanations made further below also apply for all embodiments described in this specification mutatis mutandis.
In another aspect, the present invention relates to a diagnostic test element for the determination of an analyte from a body fluid sample comprising the composition of the present invention and a carrier.
The term "carrier" as used in the context of the present invention refers to a solid support onto which the composition of the present invention is applied. Preferably, the composition can be immobilized on the carrier. Moreover, it is also envisaged that the composition can be spatially arranged on the carrier. The carrier must be arranged in a manner as to allow for the detection of the change of the at least one measurable property of the indicator reagent, i.e., it preferably does not comprise components or a spatial arrangement which would interfere with the detection of the at least one measurable property. Suitable carriers may comprise vials containing the composition of the present invention, e.g., vials arranged in a well-plate format. Other assays may apply optical waveguides or semiconductor plates. Preferred carriers, however, are those used for test strips. Said test stripes usually comprise one or more layers forming a solid carrier.

The term "body fluid sample" as used herein refers to all body fluids known or suspected to comprise the analyte to be determined. Preferred body fluids known to comprise a plurality of diagnostically relevant analytes are blood including whole blood, plasma and serum, urine, saliva, liquor, synovial liquid, and sudor. More preferably, the body fluid sample in accordance with the present invention is a whole blood sample.

The term "analyte" as used herein refers to a biological molecule present in the body fluid sample, the presence, absence or amount of which shall be determined in accordance with the present invention. Since the determination described herein is based on the enzymatic activity of a dehydrogenase, it will be understood that said analyte is a substrate of the dehydrogenase comprised by the composition. Preferred analytes envisaged to be determined in accordance with the present invention, e.g., by the aforementioned diagnostic test element are glucose, maltose, mannose, galactose, glutamate, glucose-6-phosphate, ethanol or lactose and, more preferably, glucose.

Preferably, the diagnostic test element of the invention comprises a test field containing said composition, wherein the test field has a sample application side onto which the body fluid sample is applied and a detection side which allows for detection of a change in a measurable property when the analyte reacts with the composition. The sample is to be applied to the sample application side and it is, preferably, envisaged that cells, such as erythrocytes present in blood samples, do not reach the detection side.

Details on such a test element and the manufacture thereof can be found in EP 0 821 234 B1 which is herewith incorporated by reference. Further test elements envisaged in accordance with the present invention are those disclosed in EP 1 035 919 B1 or EP 1 035 920 B1, the respective disclosure content of which is herewith incorporated by reference.

The test field of the diagnostic test element of the invention comprises, preferably, a transparent foil onto which one, more preferably more than one film layer are applied. The film layers of the diagnostic test element according to the invention are produced from dispersions or emulsions of polymeric film formers. Dispersion film formers contain microscopic polymer particles which are insoluble in the carrier liquid (usually water) and are finely dispersed in the carrier liquid. If the liquid is removed by evaporation during film formation then the particles come closer and finely touch one another. The large forces which occur in this process and the gain in surface energy which accompanies the film formation results in the particles growing into a substantially closed film layer. Alternatively, it is also possible to use an emulsion of the film former in which this is dissolved in a solvent. The dissolved polymer is emulsified in a carrier liquid which is immiscible with the solvent. Polyvinyl esters, polyvinyl acetates, polyacrylic esters, polymethacrylic acid, polyacrylamides, polyamides and polystyrene are particularly suitable as polymers for such film formers. In addition to homopolymers mixed polymerizates are also suitable such as of butadiene, styrene or maleic acid ester.

By adding a swelling agent that swells well (i.e., a substance which increases its volume when it takes up water) one does not only obtain layers which can be penetrated relatively rapidly by sample liquid but have good cell, e.g., erythrocyte and additionally also blood pigment, separation properties despite this opening effect of the swelling agent. The swelling properties should be so good that for a test in which the change of the at least one measurable property is mainly dependent on the penetration of the sample liquid through the layer, the change of the measurable property is measurable after a maximum of one minute. Especially suitable swelling agents have proven to be methyl vinyl ether maleic acid anhydride copolymer, xanthan gum and methyl vinyl ether maleic acid copolymer.

Single layer layouts of diagnostic test elements are known from the prior art, see, e.g., EP 1 566 637 and EP 1 780 288. More preferred two-layer layouts, preferably, comprise a first and a second film layer resting on top of one another in this order. It is important that the first layer located on the transparent foil scatters light considerably less than the overlying second layer. The non-coated side of the transparent foil is referred to as the detection side and the side of the second layer which is opposite to the side with which the second layer rests on the first layer is referred to as the sample application side. The two so-called film layers are located on a transparent foil in the test field of the diagnostic test carrier according to the invention. For this those plastic foils come into consideration which are impermeable to liquid. Polycarbonate foil has proven to be particularly suitable. The two film layers can be produced from coating compounds which contain the same polymeric film formers or they can be produced from coating compounds which contain different polymeric film formers. Whereas the first layer contains a swelling agent and optionally a weakly light scattering filler, the second layer requires a swelling agent and in any case at least one pigment that scatters light strongly. In addition the second layer can also contain non-porous fillers as well as porous fillers.

In the case of a single-layer film, the composition of the present invention is comprised in said single layer. In the case of multi-layer (i.e., more than one layer) layouts, it is possible that the composition of the invention is comprised in one film layer, preferably, the first film layer. However, it is also possible that the composition of the present invention is present in two or more film layers.

In order to optimize the test field in the diagnostic test carrier according to the invention, it has proven to be particularly advantageous when all film layers, preferably both layers in the case of two-layer layouts, do contain a non-haemolyzing wetting agent. Neutral, i.e., non-charged wetting agents are particularly preferred for this. N-octanoyl-N-methyl glucamide is most particularly preferred.

In order to produce a two-layer test field of a diagnostic test element according to the invention, the respective film layers are each produced successively from a homogeneous dispersion of the said components. For this, the transparent foil is used as a base to form the coating compound for the first film layer. After the coating compound for the first film layer has been applied with a particular layer thickness, the layer is dried. Afterwards the coating compound for the second layer is applied to this layer also with a thin layer thickness and dried. The test field produced in this manner can be mounted on a supporting layer for better handling, those materials coming into consideration for such a layer which do not take up the liquid to be examined. These are so-called non-absorptive materials, plastic foils for example made of polystyrene, polyvinyl chloride, polyester, polycarbonate or polyamide being particularly preferred. Metal foils or glass are suitable as further supporting materials.

In a preferred embodiment of the test element according to the invention, the detection side of the test field which is to be observed and measured for a change in at least one optical property of the indicator reagent should be visible through the supporting layer in order to determine the analyte to be detected in the body sample. This can be achieved by a transparent supporting layer. However, it is also possible that the supporting layer has a perforation which is covered by the detection side of the test field. The detection side is then visible through the perforation. Preferably, in the diagnostic test element according to the invention there is a hole in the supporting layer below the detection side of the test field through which the detection side of the test field can be observed. The hole has a somewhat smaller diameter than the smallest linear dimension of the test field so that the test field outside the hole rests on the supporting layer and can be attached there.

In another preferred embodiment of the test element according to the present invention, the test element comprises a capillary canal. Such a layout is known from the prior art, e.g., from EP 1 035 921. In a further preferred embodiment, the test element according to the present invention is produced from a test element band, see e.g., EP 1 593 434.

The present invention further relates to a method for the manufacture of a diagnostic test element comprising the step of generating a composition according to the present invention on a solid carrier.

In an embodiment of the said method of the invention, said generating comprises the steps of:
(i) applying a composition comprising the components (a) to (e) of the composition of the invention and a solvent (i.e., a composition comprising the components in a dissolved state rather than being dry) to a test field on the carrier; and
(ii) removing the said solvent from the composition;
or
(i) applying a composition comprising components (a), (b), (d) and (e) of the composition of the invention and a solvent (i.e., a composition comprising the components in a dissolved state rather than being dry) to a test field on the carrier in a first layer;
(ii) removing the said solvent from the composition of the first layer;
(iii) applying a composition comprising components (c) to (e) of the composition of the invention and a solvent (i.e., a composition comprising the components in a dissolved state rather than being dry) in a second layer on the first layer; and
(iv) removing the said solvent from the composition of the second layer;
or
(i) applying a composition comprising components (a), (b), (d) and (e) of the composition of the invention and a solvent (i.e,. a composition comprising the components in a dissolved state rather than being dry) to a test field on the carrier in a first layer;
(ii) removing the said solvent from the composition of the first layer;
(iii) applying a composition comprising components (b) to (e) of the composition of the invention and a solvent (i.e., a composition comprising the components in a dissolved state rather than being dry) in a second layer on the first layer; and
(iv) removing the said solvent from the composition of the second layer;
or
(i) applying a composition comprising components (a), (d) and (e) of the composition of the invention and a solvent (i.e., a composition comprising the components in a dissolved state rather than being dry) to a test field on the carrier in a first layer;
(ii) removing the said solvent from the composition of the first layer;
(iii) applying a composition comprising components (b) to (e) of the composition of the invention and a solvent (i.e., a composition comprising the components in a dissolved state rather than being dry) in a second layer on the first layer; and
(iv) removing the said solvent from the composition of the second layer;
or
(i) applying a composition comprising components (c) to (e) of the composition of the invention and a solvent (i.e., a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of the invention and a solvent (i.e., a composition comprising the components in a dissolved state rather than being dry) in a second layer on the first layer; and
(iv) removing the said solvent from the composition of the second layer.
or
(i) applying a composition comprising components (b) to (e) of the composition of the invention and a solvent (i.e., a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of the invention and a solvent (i.e., a composition comprising the components in a dissolved state rather than being dry) in a second layer on the first layer; and
(iv) removing the said solvent from the composition of the second layer.
or
(i) applying a composition comprising components (b) to (e) of the composition of the invention and a solvent (i.e., a composition comprising the components in a dissolved state rather than being dry) to a test field on the carrier in a first layer;
(ii) removing the said solvent from the composition of the first layer;
(iii) applying a composition comprising components (a), (d) and (e) of the composition of the invention and a solvent (i.e., a composition comprising the components in a dissolved state rather than being dry) in a second layer on the first layer; and
(iv) removing the said solvent from the composition of the second layer.

Preferably, the composition is applied to a detection layer in the test field. The detection layer can be generated in particular by means of at least one wet chemical process, more particularly from one or more dispersions, preferably aqueous dispersions of the composition of the present invention. Such layer-forming processes from one or more dispersions are known in principle to a person skilled in the art, and reference can be exemplarily made in turn to, for example, the abovementioned prior art, more particularly EP 0 821 234 B1.

The solvent can be removed from the composition after application of the said composition to the test field of the test element by all techniques known for removing solvents including heat treatment, evaporation or freeze-drying. Preferably, said solvent in step (ii) is substantially removed by heat treatment.

Also preferably, said pyridine-carboxylic acid or a derivative thereof reduces the decrease of the enzymatic activity of the at least one enzyme in the composition and, in particular, during the substantial removal of the solvent in step (ii) which is achieved, e.g., by heat treatment and during the maintenance of the composition under dry conditions on the test element.
The present invention also contemplates, in principle, the use of at least one pyridine-carboxylic acid for preventing a reduction of the enzymatic activity of at least one enzyme in a composition under dry or humid conditions, wherein said composition comprises a dehydrogenase, a redox cofactor, an agent capable of eliciting a change in at least one measurable property or an indicator reagent in the presence of redox equivalents, and an indicator reagent.

Preferably, said at least one carboxylic acid in the composition under dry or under humid conditions is comprised in a diagnostic test element, preferably a diagnostic test element as specified above in detail.
The present invention also relates to a method for determining the presence or amount of an analyte in a body fluid sample comprising the steps of:
(a) contacting the diagnostic test element of the invention with a body fluid suspected to comprise the analyte under conditions suitable for transforming the at least one enzyme to the reconstituted state;
(b) measuring a change in at least one measurable property of the indicator reagent in the wetted composition comprising the at least one enzyme in the reconstituted state on the diagnostic test element, whereby the presence or amount of the analyte in the body fluid sample is determined.
As set forth elsewhere herein already, depending on the substrate specificity of the dehydrogenase to be used in the composition on the diagnostic test element, different analytes can be determined by the method of the invention.
Contacting as used herein means that the body fluid sample is applied to the carrier in a manner as to allow for physical contact of the composition of the invention comprised by the carrier and the body fluid sample. In particular, contacting is carried out for a time and under conditions being sufficient for allowing the dehydrogenase to be reconstituted, i.e., wetted and dissolved, and, thus, to become biologically active. Suitable conditions depend on the diagnostic carrier and are known in the art. The body fluid sample applied to the test element, preferably, has a volume of less than 2 microliters, more particularly of less than 1 microliter. In a preferred embodiment, the body fluid sample applied to the test element, preferably, has a volume of less than 20 microliters, more preferably of less than 3 microliters.

Upon reconstitution of the said biologically active dehydrogenase, the enzyme shall bind to its substrate, i.e., the analyte comprised in the body fluid sample, and convert it into the respective product and redox equivalents. The redox equivalents generated by the dehydrogenase allow for determining the dehydrogenase activity since the redox equivalents generated by the enzymatic conversion catalyzed by the dehydrogenase are transferred by the agent capable of eliciting a change in at least one measurable property of the indicator reagent in the presence of redox equivalents in the composition comprised to the indicator reagent. The change in the at least one measurable property of the indicator reagent can then be measured. Depending on the diagnostic test element, the measurement of the change of the measurable property can be achieved by different techniques known in the art and described, e.g., in WO2012010454A1; WO2007115732A1, and US6055060. Preferably, the measurable property is an optical property and the detector includes at least one light source for illuminating test element and at least one optically sensitive element adapted to determine detection light from Said test element. For detecting the change of an optical property such as color, also a spatially resolving optical detector may be used. A spatially resolving optical detector is to be understood to mean an optical detector which has a multiplicity of optical sensors which are able to record regions of the detection side of the detection layer, e.g., a CCD chip and/or CMOS chip. In addition, the spatially resolving optical detector can comprise at least one optical element for imaging the detection side and/or the detection layer onto an image-sensitive surface of the spatially resolving optical detector.

A change in at least one measurable property measured by the method described above shall be indicative for the presence of the analyte. It will be understood by the skilled artisan that in order to determine the amount of an analyte, it might be necessary to compare the extent of the change of the measurable property. To this end, it might be, furthermore, necessary to compare a detected signal accompanying the measurable change to signals accompanying measurable changes elicited by known amounts of analytes, i.e., calibration signals and / or reference values. How such a calibration can be established is well known to the skilled artisan.

The term "amount" as used herein refers to the absolute or relative amount of analyte present in a sample applied to the diagnostic test element. A relative amount preferred according to the present invention is the concentration, i.e., the amount in relation to the volume.

### FIGURES

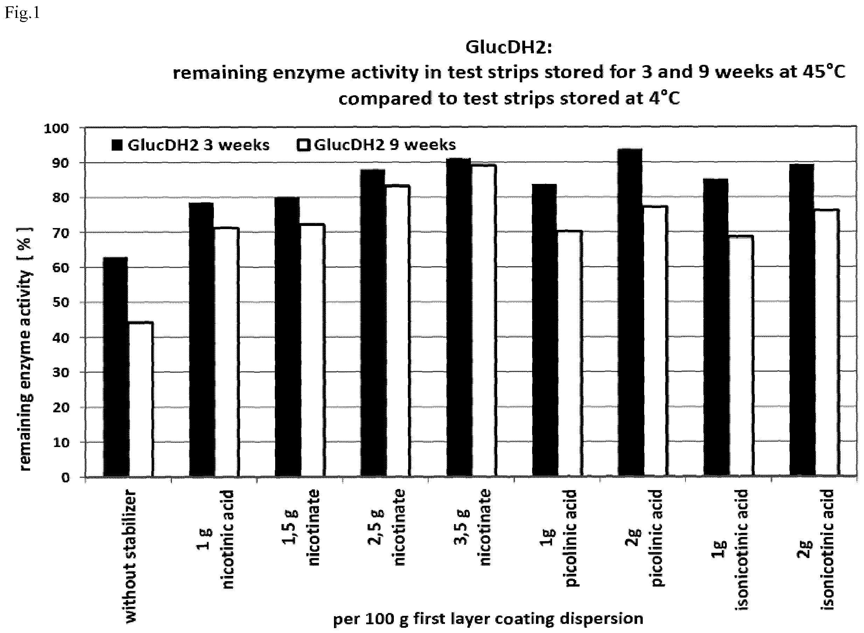

**Fig. 1****:** Stabilizing effect of nicotinic acid derivatives on Glucosedehydrogenase Shown is the remaining activity of Glucosedehydrogenase mutant 2 (ordinate) after storage for three and nine weeks at 45°C (abscissa) in a composition according to the present invention (coating on test strip), as compared to the remaining activity after storage at 4°C (=100%).

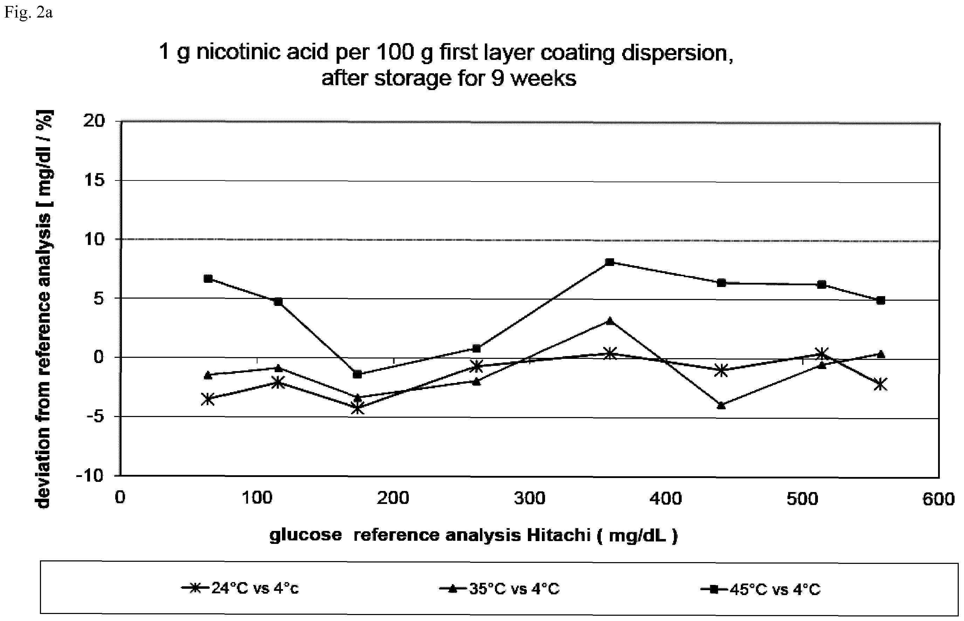

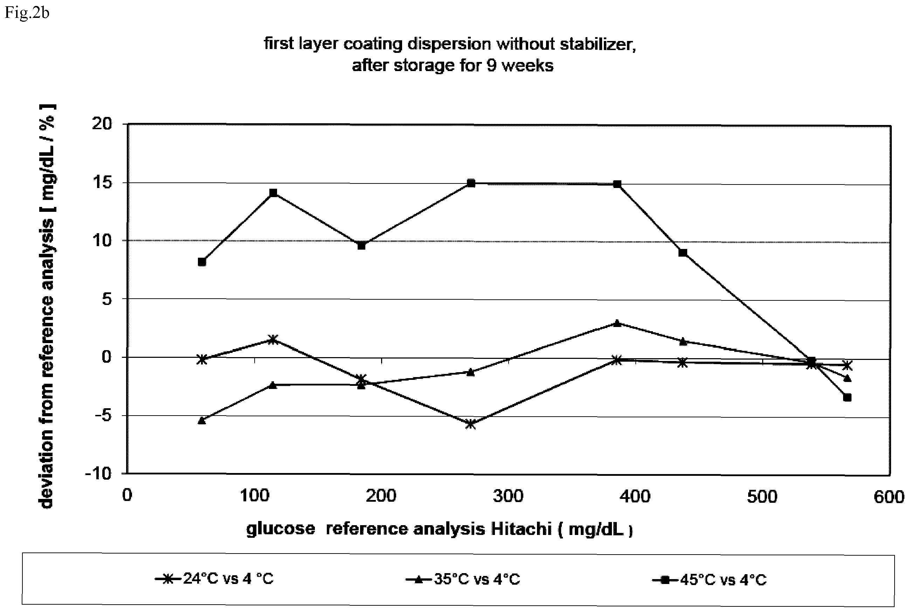

**Fig. 2****:** Glucose concentration vs. readings from test elements stored at various temperatures normalized against readings from test elements stored at 4°C. 2a) Test elements produced with 1g nicotinic acid per 100 g first layer coating dispersion. 2b) Test elements produced without stabilizer added to the first layer coating dispersion.

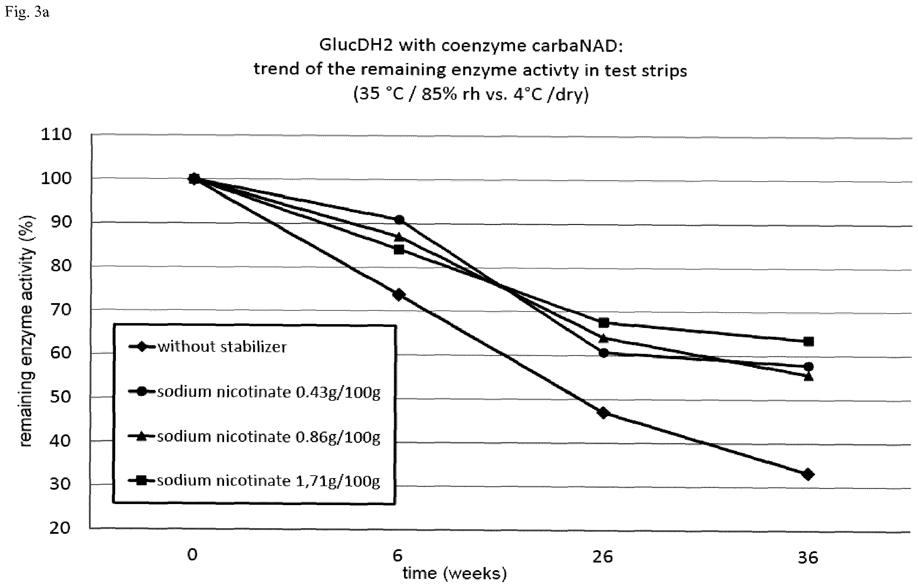

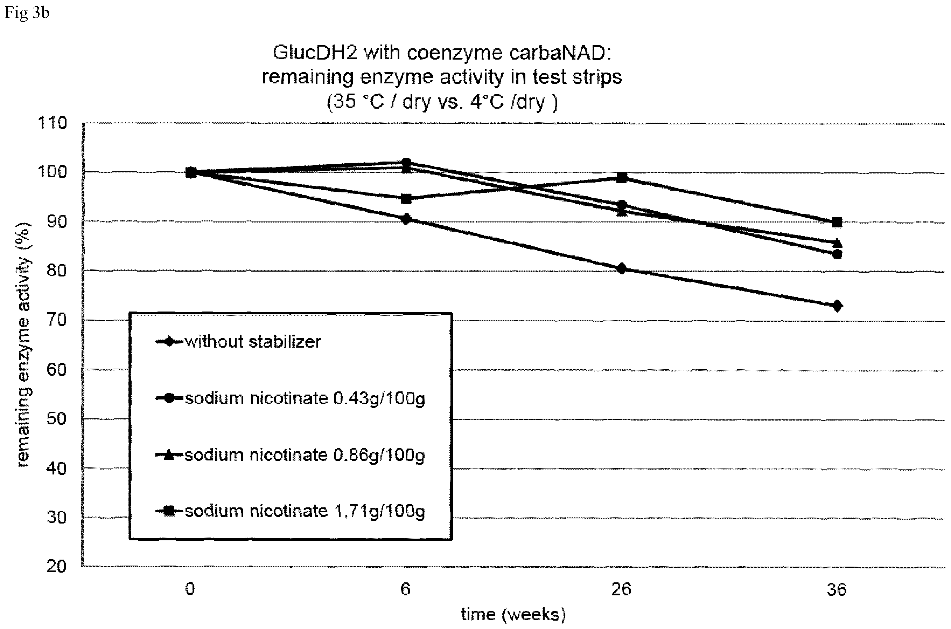

**Fig. 3****:** Stabilizing effect of nicotinic acid at 35°C under dry and under humid storage conditions
   Shown is the time course of remaining activity stored in a composition as described herein (coating on test strips) at 35°C under humid (3a) or under dry (3b) conditions, as compared to the residual activity after storage at 4°C und dry conditions (=100%).

### EXAMPLES

### Example 1:

Different reaction films for determining glucose levels were generated and coated on a foil essentially as described in EP 0 821 234, Example 1. The formulation of the composition is shown in the following table 1.

**Table 1: Components per 100 g of the first coating film prior to drying**

| | | | | | |
|---|---|---|---|---|---|
| Glucosedehydrogenase from Bacillus subtilis | 1.09 g | 1.09 g | 1.09 g | 1.09 g | 1.09 g |
| Diaphorase from Bacillus subtilis | 0.77 g | 0.77 g | 0.77 g | 0.77 g | 0.77 g |
| NAD | 0.58 g | 0.58 g | 0.58 g | 0.58 g | 0.58 g |
| Na/KPhosphate buffer or HEPES | 0.35g | 0.35g | 0.35g | 0.35g | 0.35g |
| **Nicotinic acid (resp. picolinic acid or isonicotinic acid)** | **0.00 g** | **1.0 g** | **1.5 g** | **2.5 g** | **3**.**5g** |
| Xanthan gum | 0.29 g | 0.29 g | 0.29 g | 0.29 g | 0.29 g |
| Silica FK 320DS | 5.80 g | 5.80 g | 5.80 g | 5.80 g | 5.80 g |
| Sodium-N-methyl-N-oleoyl-taurate | 0.03 g | 0.03 g | 0.03 g | 0.03 g | 0.03 g |
| N-Octanoyl-N-methyl-glucamide | 0.17 g | 0.17 g | 0.17 g | 0.17 g | 0.17 g |
| Polyvinylpyrrolidone | 0.86 g | 0.86 g | 0.86 g | 0.86 g | 0.86 g |
| Tetraethylammoniumchloride | 0.07 g | 0.07 g | 0.07 g | 0.07 g | 0.07 g |
| 2,18-Phosphormolybdenic acid hexasodium salt | 0.33 g | 0.33 g | 0.33 g | 0.33 g | 0.33 g |
| Polyvinylpropionate-Dispersion (50 Gew.-% in water) | 5.00 g | 5.00 g | 5.00 g | 5.00 g | 5.00 g |
| K3[Fe(CN)6] | 0.01 g | 0.01 g | 0.01 g | 0.01 g | 0.01 g |
| 2-Methyl-2 butanol | 1.00 g | 1.00 g | 1.00 g | 1.00 g | 1.00 g |
| add water up to 100g | | | | | |

A pH of 6.8 was adjusted and the composition was coated as a film (about 120 micrometer) on a polycarbonate foil (125 micrometer). The coated composition was subsequently dried at 50°C.

**Table 2: Components of the second coating film**

| | |
|---|---|
| Gantrez | 1.47 g |
| sodium-N-methyl-N-oleoyl-taurat | 0.03 g |
| PVP K25 | 2.01 g |
| Mega 8 | 0.37 g |
| Tetraethylammoniumchloride | 0.45 g |
| silica FK 320DS | 2.00 g |
| Titandioxide E 171 | 22.00 g |
| Polyvinylpropionate-Dispersion (50 w% in water) | 6.25 g |
| Bis-(2-hydroxyethyl)-(4-hydroximinocyclohexa-2,5-dienylidin)-ammoniumchloride | 0.48 g |
| 2,18-Phosphormolybdenic acid hexasodium salt | 1.41 g |
| K3[Fe(CN)6] | 0.01 g |
| 2-Methyl-2 butanol | 1.00 g |
| add water up to 100g | |

A pH of 6.8 was adjusted and the composition was coated as a second film (about 25 micrometer) onto the first film coated on the foil. The coated composition was subsequently dried at 50°C. Test strips for glucose determination were generated as described in EP 0 821 234, sections ([0063 - 0067]).

### Example 2: Determination of the enzymatic activity in test elements after storage

Test elements were stored in plastic vials in the presence of a desiccant at 4 °C and 45°C for 3 and 9 weeks.
In a subsequent step, the reagent pads of five test elements were eluted by ultra-sonication using elution buffer. In the supernatant, enzymatic activity was determined and mean value calculated.
Elution buffer: Tris/HCl, NaCl, NAD; pH 8.5
Detection technique: UV-detection at 340 nm

Results are shown in Fig. 1. The Figure shows that there is a significant stabilizing effect (more than 10%) with nicotinic acid, picolinic acid and isonicotinic acid.

### Example 3: Determination of the test element function after different storage conditions

Test elements were stored in plastic vials in the presence of a drying agent for 9 weeks at 4°C; 24°C; 35°C and 45°C. Test elements were used to determine blood glucose levels in a plurality of venous blood samples. The samples were measured with a reference method (Hitachi) in parallel. Results were normalized with respect to the reference samples. Deviation of the indicated glucose values from reference is shown in Fig. 2a (with nicotinic acid) and 2b (without added stabilizer). Each data point is a mean from 10 test elements.

Test elements without added stabilizer stored at 45°C show higher glucose values in the range of 100 - 400 mg/dL glucose (deviations > 10 %), whereas test elements with nicotinic acid show deviations within ± 8%.

### Example 4 Stabilization of Gluc-DH Mut2 in the presence of coenzyme carbaNAD.

Test elements with added nicotinic acid were prepared and analog to example 1, but with adding coenzyme carbaNAD ('carbaNAD', for ref. WO2007012494A1) instead of NAD (same amount on molar basis).
As carbaNAD itself is much more stable than NAD, more challenging conditions for the test elements were chosen: test elements were stored
a) in open vials at 85% r.h. and 35°C,
b) in closed vials with desiccant at 35°C.

Analysis of enzymatic activity was the same as in example 2.
Results are shown in Fig. 3a (open vials at high humidity) and 3b (closed vials).
The Figure shows that there is a significant stabilizing effect with increasing amounts of nicotinic acid.

## Claims

1. A dry composition comprising the components:
(a) a dehydrogenase;
(b) a redox cofactor;
(c) an agent capable of eliciting a change in at least one measurable property of an indicator reagent in the presence of redox equivalents;
(d) an indicator reagent; and
(e) at least one pyridine-carboxylic acid or a salt thereof,
wherein said pyridine-carboxylic acid is a pyridine-monocarboxylic acid or a pyridine-dicarboxylic acid.

2. The composition of claim 1, wherein said dehydrogenase is a glucose dehydrogenase, preferably selected from the group consisting of: glucose dehydrogenase (EC number 1.1.1.47), quinoprotein glucose dehydrogenase (EC number 1.1.5.2), in particular, Pyrrolo quinoline quinone (PQQ)-dependent glucose dehydrogenase (EC number 1.1.5.2), glucose-6-phosphate dehydrogenase (EC number 1.1.1.49), nicotinamide adenine dinucleotide (NAD)-dependent glucose dehydrogenase (EC number 1.1.1.119) and flavin adenine dinucleotide (FAD)-dependent glucose dehydrogenase (EC number 1.1.99.10), and enzymatically active mutants thereof.

3. The composition of claim 1 or 2, wherein said pyridine-carboxylic acid is selected from the group consisting of: pyridine-3-carboxylic acid (niacin, nicotinic acid), pyridine-4-carboxylic acid (isonicotinic acid), pyridine-2-carboxylic acid (picolinic acid), and the sodium and potassium salts thereof.

4. The composition of any one of claims 1 to 3, wherein said agent capable of eliciting a change in the at least one measurable property in the presence of redox equivalents is capable of transferring said redox equivalents from the redox cofactor to the indicator reagent.

5. The composition of claim 4, wherein said agent capable of eliciting a change in the at least one measurable property in the presence of redox equivalents is (i) a diaphorase, preferably, a lipoamide deydrogenase or a NADH dehydrogenase, (ii) a phenazine, preferably, phenazinethosulfate, phenazinmethosulfate, 1-(3-carboxypropoxy)-5-ethylphenaziniumtrifluoromethansulfonate or 1-methoxyphenazinmethosulfate, (iii) a nitrosoaniline, preferably, [(4-nirosophenyl)imino]dimethanol-hydrochloride, or (iv) a chinone, preferably, phenanthrenechinone, phenanthrolinchinone or benzo[h]-chinolinchinone.

6. The composition of any one of claims 1 to 5, wherein said redox cofactor is selected from the group consisting of: carbaNAD, NAD, FAD, and PQQ.

7. The composition of any one of claims 1 to 6, wherein the measurable property is an optical property.

8. A diagnostic test element for the determination of an analyte from a body fluid sample comprising the composition of any one of claims 1 to 7 on a carrier.

9. The diagnostic test element of claim 8, wherein said carrier comprises a test field containing said composition, wherein the test field has a sample application side onto which the body fluid sample is applied and a detection side which allows for detection of the change in at least one measurable property of the reagent when the analyte reacts with the composition.

10. A method for the manufacture of a diagnostic test element comprising the step of generating a composition according to any one of claims 1 to 7 on a carrier.

11. The method of claim 10, wherein said generating comprises the steps of:
(i) applying a composition comprising the components (a) to (e) of the composition of any one of claims 1 to 7 and a solvent (i.e., a composition comprising the components in a dissolved state rather than being dry) to a test field on the carrier; and
(ii) removing the said solvent from the composition;
or
(i) applying a composition comprising components (a), (b), (d) and (e) of the composition of any one of claims 1 to 7 and a solvent (i.e., a composition comprising the components in a dissolved state rather than being dry) to a test field on the carrier in a first layer;
(ii) removing the said solvent from the composition of the first layer;
(iii) applying a composition comprising components (c) to (e) of the composition of any one of claims 1 to 7 and a solvent (i.e., a composition comprising the components in a dissolved state rather than being dry) in a second layer on the first layer; and
(iv) removing the said solvent from the composition of the second layer;
or
(i) applying a composition comprising components (a), (b), (d) and (e) of the composition of any one of claims 1 to 7 and a solvent (i.e., a composition comprising the components in a dissolved state rather than being dry) to a test field on the carrier in a first layer;
(ii) removing the said solvent from the composition of the first layer;
(iii) applying a composition comprising components (b) to (e) of the composition of any one of claims 1 to 7 and a solvent (i.e., a composition comprising the components in a dissolved state rather than being dry) in a second layer on the first layer; and
(iv) removing the said solvent from the composition of the second layer;
or
(i) applying a composition comprising components (a), (d) and (e) of the composition of any one of claims 1 to 7 and a solvent (i.e., a composition comprising the components in a dissolved state rather than being dry) to a test field on the carrier in a first layer;
(ii) removing the said solvent from the composition of the first layer;
(iii) applying a composition comprising components (b) to (e) of the composition of any one of claims 1 to 7 and a solvent (i.e., a composition comprising the components in a dissolved state rather than being dry) in a second layer on the first layer; and
(iv) removing the said solvent from the composition of the second layer;
or
(i) applying a composition comprising components (c) to (e) of the composition of any one of claims 1 to 7 and a solvent (i.e., a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of any one of claims 1 to 7 and a solvent (i.e., a composition comprising the components in a dissolved state rather than being dry) in a second layer on the first layer; and
(iv) removing the said solvent from the composition of the second layer.
or
(i) applying a composition comprising components (b) to (e) of the composition of any one of claims 1 to 7 and a solvent (i.e., a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of any one of claims 1 to 7 and a solvent (i.e., a composition comprising the components in a dissolved state rather than being dry) in a second layer on the first layer; and
(iv) removing the said solvent from the composition of the second layer.
or
(i) applying a composition comprising components (b) to (e) of the composition of any one of claims 1 to 7 and a solvent (i.e., a composition comprising the components in a dissolved state rather than being dry) to a test field on the carrier in a first layer;
(ii) removing the said solvent from the composition of the first layer;
(iii) applying a composition comprising components (a), (d) and (e) of the composition of any one of claims 1 to 7 and a solvent (i.e., a composition comprising the components in a dissolved state rather than being dry) in a second layer on the first layer; and
(iv) removing the said solvent from the composition of the second layer.

12. The dry composition of any one of claims 1 to 7, the diagnostic test element of claim 8 or 9, or the method of claim 10 or 11, wherein the pyridine-carboxylic acid reduces a decrease of the enzymatic activity of at least one enzyme in the composition under dry and / or under humid conditions.

13. Use of at least one pyridine-carboxylic acid for reducing a decrease of the enzymatic activity of at least one enzyme in a composition under dry and/or under humid conditions, wherein said composition comprises a dehydrogenase, a redox cofactor, an agent capable of eliciting a change in at least one measurable property of an indicator reagent in the presence of redox equivalents, and an indicator reagent,
wherein said pyridine-carboxylic acid is a pyridine-monocarboxylic acid or a pyridine-dicarboxylic acid.

14. The use of claim 13, wherein said at least one pyridine-carboxylic acid in the composition under dry conditions is comprised in a diagnostic test element, preferably, a diagnostic test element according to claim 8 or 9.

15. A method for determining the presence or amount of an analyte in a body fluid sample comprising the steps of:
(a) contacting the diagnostic test element of claim 8 or 9 with a body fluid suspected to comprise the analyte under conditions suitable for transforming the at least one enzyme to the reconstituted state;
(b) measuring a change in at least one measurable property of the indicator reagent in the wetted composition comprising the at least one enzyme in the reconstituted state on the diagnostic test element, whereby the presence or amount of the analyte in the body fluid sample is determined.

## Patentansprüche

1. Trockene Zusammensetzung umfassend die Komponenten:
(a) eine Dehydrogenase;
(b) einen Redox Ko-Faktor;
(c) ein Mittel, das in der Lage ist, eine Änderung in mindestens einer messbaren Eigenschaft eines Indikatorreagenzes in Gegenwart von Redoxäquivalenten hervorzurufen;
(d) ein Indikatorreagenz; und
(e) mindestens eine Pyridin-Carbonsäure oder ein Salz davon;
wobei die Pyridin-Carbonsäure eine Pyridin-Monocarbonsäure oder eine Pyridin-Dicarbonsäure ist.

2. Zusammensetzung nach Anspruch 1, wobei die Dehydrogenase eine Glucose Dehydrogenase ist, bevorzugt ausgewählt aus der Gruppe bestehend aus: Glucose Dehydrogenase (EC Nummer 1.1.1.47), Quinoprotein Glucose Dehydrogenase (EC Nummer 1.1.5.2), insbesondere, Pyrrolo Quinoline Quinone (PQQ)-abhängige Glucose Dehydrogenase (EC Nummer 1.1.5.2), Glucose-6-Phosphat Dehydrogenase (EC Nummer 1.1.1.49), Nikotinamid Adenin Dinukleotid (NAD)-abhängige Glucose Dehydrogenase (EC Nummer 1.1.1.119) und Flavin Adenin Dinukleotid (FAD)-abhängige Glucose Dehydrogenase (EC Nummer 1.1.99.10), und enzymatisch active Mutanten davon.

3. Zusammensetzung nach Anspruch 1 oder 2, wobei die Pyridin-Carbonsäure ausgewählt ist aus der Gruppe bestehend aus: Pyridin-3-Carbonsäure (Niacin, Nikotinsäure), Pyridin-4-Carbonsäure (Isonikotinsäure), Pyridin-2-Carbonsäure (Picolinsäure), und deren Natrium- und Kaliumsalze.

4. Zusammensetzung nach einem der Ansprüche 1 bis 3, wobei das Mittel, das in der Lage ist, eine Änderung in mindestens einer messbaren Eigenschaft eines Indikatorreagenzes in Gegenwart von Redoxäquivalenten hervorzurufen, in der Lage ist, die Redoxäquivalente von dem Redox Ko-Faktor zu dem Indikatorreagenz zu übertragen.

5. Zusammensetzung nach Anspruch 4, wobei das Mittel, das in der Lage ist, eine Änderung in mindestens einer messbaren Eigenschaft eines Indikatorreagenzes in Gegenwart von Redoxäquivalenten hervorzurufen (i) eine Diaphorase, bevorzugt, eine Lipoamid Deydrogenase oder eine NADH Dehydrogenase, (ii) ein Phenazin, bevorzugt, Phenazinethosulfat, Phenazinmethosulfat, 1-(3-Carboxypropoxy)-5-Ethylphenaziniumtrifluoromethansulfonat oder 1-Methoxyphenazinmethosulfat, (iii) ein Nitrosoanilin, bevorzugt, [(4-Nitrosophenyl)Imino]Dimethanol-Hydrochlorid, oder (iv) ein Chinone, bevorzugt, Phenanthrenechinone, Phenanthrolinchinone oder Benzo[h]-Chinolinchinone ist.

6. Zusammensetzung nach einem der Ansprüche 1 bis 5, wobei der Redox Ko-Faktor ausgewählt ist aus der Gruppe bestehend aus: CarbaNAD, NAD, FAD, und PQQ.

7. Zusammensetzung nach einem der Ansprüche 1 bis 6, wobei die messbare Eigenschaft eine optische Eigenschaft ist.

8. Diagnostisches Testelement zur Bestimmung eines Analyten aus einer Körperflüssigkeitsprobe umfassend die Zusammensetzung nach einem der Ansprüche 1 bis 7 auf einem Träger.

9. Diagnostisches Testelement nach Anspruch 8, wobei der Träger ein Testfeld umfasst, das die Zusammensetzung enthält, wobei das Testfeld eine Probeanwendungsseite aufweist, auf die die Körperflüssigkeitsprobe aufgebracht wird, und eine Erkennungsseite, die die Erkennung der Änderung in mindestens einer messbaren Eigenschaft des Reagenzes ermöglicht, wenn der Analyt mit der Zusammensetzung reagiert.

10. Verfahren zur Herstellung eines diagnostischen Testelementes umfassend den Schritt des Erzeugens einer Zusammensetzung nach einem der Ansprüche 1 bis 7 auf einem Träger.

11. Verfahren nach Anspruch 10, wobei das Erzeugen die folgenden Schritte umfasst:
(i) Aufbringen einer Zusammensetzung, umfassend die Komponenten (a) bis (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) auf ein Testfeld auf dem Träger; und
(ii) Entfernen des Lösungsmittels aus der Zusammensetzung;
oder
(i) Aufbringen einer Zusammensetzung, umfassend die Komponenten (a), (b), (d) und (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) auf ein Testfeld auf dem Träger in einer ersten Schicht;
(ii) Entfernen des Lösungsmittels aus der Zusammensetzung der ersten Schicht;
(iii) Aufbringen einer Zusammensetzung, umfassend die Komponenten (c) bis (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) in einer zweiten Schicht auf der ersten Schicht; und
(iv) Entfernen des Lösungsmittels aus der Zusammensetzung der zweiten Schicht;
oder
(i) Aufbringen einer Zusammensetzung, umfassend die Komponenten (a), (b), (d) und (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) auf ein Testfeld auf dem Träger in einer ersten Schicht;
(ii) Entfernen des Lösungsmittels aus der Zusammensetzung der ersten Schicht;
(iii) Aufbringen einer Zusammensetzung, umfassend die Komponenten (b) bis (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) in einer zweiten Schicht auf der ersten Schicht; und
(iv) Entfernen des Lösungsmittels aus der Zusammensetzung der zweiten Schicht;
oder
(i) Aufbringen einer Zusammensetzung, umfassend die Komponenten (a), (d) und (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) auf ein Testfeld auf dem Träger in einer ersten Schicht;
(ii) Entfernen des Lösungsmittels aus der Zusammensetzung der ersten Schicht;
(iii) Aufbringen einer Zusammensetzung, umfassend die Komponenten (b) bis (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) in einer zweiten Schicht auf der ersten Schicht; und
(iv) Entfernen des Lösungsmittels aus der Zusammensetzung der zweiten Schicht
oder
(i) Aufbringen einer Zusammensetzung, umfassend die Komponenten (c) bis (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) auf ein Testfeld auf dem Träger in einer ersten Schicht;
(ii) Entfernen des Lösungsmittels aus der Zusammensetzung der ersten Schicht;
(iii) Aufbringen einer Zusammensetzung, umfassend die (a), (b), (d) und (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) in einer zweiten Schicht auf der ersten Schicht; und
(iv) Entfernen des Lösungsmittels aus der Zusammensetzung der zweiten Schicht;.
oder
(i) Aufbringen einer Zusammensetzung, umfassend die Komponenten (b) bis (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) auf ein Testfeld auf dem Träger in einer ersten Schicht;
(ii) Entfernen des Lösungsmittels aus der Zusammensetzung der ersten Schicht;
(iii) Aufbringen einer Zusammensetzung, umfassend die Komponenten (a), (b), (d) und (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) in einer zweiten Schicht auf der ersten Schicht; und
(iv) Entfernen des Lösungsmittels aus der Zusammensetzung der zweiten Schicht.
oder
(i) Aufbringen einer Zusammensetzung, umfassend die Komponenten (b) bis (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) auf ein Testfeld auf dem Träger in einer ersten Schicht;
(ii) Entfernen des Lösungsmittels aus der Zusammensetzung der ersten Schicht;
(iii) Aufbringen einer Zusammensetzung, umfassend die Komponenten (a), (d) und (e) der Zusammensetzung nach einem der Ansprüche 1 bis 7 und ein Lösungsmittel (d.h., eine Zusammensetzung, die die Komponenten in einem gelösten Zustand enthält, anstatt trocken zu sein) in einer zweiten Schicht auf der ersten Schicht; und
(iv) Entfernen des Lösungsmittels aus der Zusammensetzung der zweiten Schicht.

12. Trockene Zusammensetzung nach einem der Ansprüche 1 bis 7, diagnostisches Testelement nach Anspruch 8 oder 9, oder Verfahren nach Anspruch 10 oder 11, wobei die Pyridin-Carbonsäure eine Abnahme der enzymatischen Aktivität von mindestens einem Enzym in der Zusammensetzung unter trockenen und/oder unter feuchten Bedingungen verringert.

13. Verwendung von mindestens einer Pyridin-Carbonsäure zur Verringerung einer Abnahme der enzymatischen Aktivität von mindestens einem Enzym in einer Zusammensetzung unter trockenen und/oder unter feuchten Bedingungen, wobei die Zusammensetzung eine Dehydrogenase, einen Redox Ko-Faktor, ein Mittel, das in der Lage ist, eine Änderung in mindestens einer messbaren Eigenschaft eines Indikatorreagenzes in Gegenwart von Redoxäquivalenten hervorzurufen, und ein Indikatorreagenz umfasst,
wobei die Pyridin-Carbonsäure eine Pyridin-Monocarbonsäure oder eine Pyridin-Dicarbonsäure ist.

14. Verwendung nach Anspruch 13, wobei die mindestens eine Pyridin-Carbonsäure in der Zusammensetzung unter trockenen Bedingungen in einem diagnostischen Testelement, bevorzugt einem diagnostischen Testelement nach Anspruch 8 oder 9, enthalten ist.

15. Verfahren zur Bestimmung des Vorhandenseins oder der Menge eines Analyten in einer Körperflüssigkeitsprobe, umfassend die Schritte:
(a) Kontaktieren des diagnostischen Testelements nach Anspruch 8 oder 9 mit einer Körperflüssigkeit, von der vermutet wird, dass sie den Analyten umfasst, unter Bedingungen, die geeignet sind, um das mindestens eine Enzym in den rekonstituierten Zustand umzuwandeln;
(b) Messen einer Änderung in mindestens einer messbaren Eigenschaft des Indikatorreagenzes in der benetzten Zusammensetzung, die das mindestens eine Enzym im rekonstituierten Zustand auf dem diagnostischen Testelement umfasst, wobei die Anwesenheit oder Menge des Analyten in der Körperflüssigkeitsprobe bestimmt wird.

## Revendications

1. Composition sèche comprenant les constituants :
(a) une déshydrogénase ;
(b) un cofacteur redox ;
(c) un agent pouvant provoquer un changement dans au moins une propriété mesurable d'un réactif indicateur en présence d'équivalents redox ;
(d) un réactif indicateur ; et
(e) au moins un acide pyridinecarboxylique ou un sel de celui-ci,
ledit acide pyridinecarboxylique étant un acide pyridinemonocarboxylique ou un acide pyridinedicarboxylique.

2. Composition selon la revendication 1, ladite déshydrogénase étant une glucose déshydrogénase, de préférence choisie dans le groupe constitué par: la glucose déshydrogénase (numéro CE 1.1.1.47), la quinoprotéine glucose déshydrogénase (numéro CE 1.1.5.2), en particulier la glucose déshydrogénase dépendant de la pyrroloquinoléinequinone (PQQ) (numéro CE 1.1.5.2), la glucose-6-phosphate déshydrogénase (numéro CE 1.1.1.49), la glucose déshydrogénase dépendant du nicotinamide-adénine dinucléotide (NAD) (numéro CE 1.1.1.119) et la glucose déshydrogénase dépendant du flavine-adénine dinucléotide (FAD) (numéro CE 1.1.99.10) et les mutants enzymatiquement actifs de celles-ci.

3. Composition selon la revendication 1 ou 2, ledit acide pyridinecarboxylique étant choisi dans le groupe constitué par : l'acide pyridine-3-carboxylique (niacine, acide nicotinique), l'acide pyridine-4-carboxylique (acide isonicotinique), l'acide pyridine-2-carboxylique (acide picolinique) et les sels de sodium et de potassium de ceux-ci.

4. Composition selon l'une quelconque des revendications 1 à 3, ledit agent pouvant provoquer un changement dans au moins une propriété mesurable en présence d'équivalents redox pouvant transférer lesdits équivalents redox du cofacteur redox au réacteur indicateur.

5. Composition selon la revendication 4, ledit agent pouvant provoquer un changement dans au moins une propriété mesurable en présence d'équivalents redox étant (i) une diaphorase, de préférence un lipoamide déshydrogénase ou une NADH déshydrogénase, (ii) une phénazine, de préférence l'éthosulfate de phénazine, le méthosulfate de phénazine, le trifluorométhanesulfonate de 1-(3-carboxypropoxy)-5-éthylphénazinium ou le méthosulfate de 1-méthoxyphénazine, (iii) une nitrosoaniline, de préférence le chlorhydrate de [(4-nitrosophényl)imino]diméthanol, ou (iv) une quinone, de préférence la phénanthrènequinone, la phénanthrolinequinone ou la benzo[h]-quinoléinequinone.

6. Composition selon l'une quelconque des revendications 1 à 5, ledit cofacteur redox étant choisi dans le groupe constitué par : carbaNAD, NAD, FAD et PQQ.

7. Composition selon l'une quelconque des revendications 1 à 6, la propriété mesurable étant une propriété optique.

8. Elément de test diagnostique pour la détermination d'un analyte d'un échantillon de fluide corporel, comprenant la composition selon l'une quelconque des revendications 1 à 7 sur un support.

9. Elément de test diagnostique selon la revendication 8, ledit support comprenant une zone de test contenant ladite composition, la zone de test présentant un côté d'application d'échantillon, sur lequel l'échantillon de fluide corporel est appliqué, et un côté de détection, qui permet la détection du changement dans au moins une propriété mesurable du réactif lorsque l'analyte réagit avec la composition.

10. Procédé pour la fabrication d'un élément de test diagnostique comprenant l'étape de génération d'une composition selon l'une quelconque des revendications 1 à 7 sur un support.

11. Procédé selon la revendication 10, ladite génération comprenant les étapes de :
(i) application d'une composition comprenant les constituants (a) à (e) de la composition selon l'une quelconque des revendications 1 à 7 et un solvant (c'est-à-dire, une composition comprenant les constituants dans un état dissous plutôt qu'à l'état sec) sur une zone de test sur le support ; et
(ii) élimination dudit solvant de la composition ; ou
(i) application d'une composition comprenant les constituants (a), (b), (d) et (e) de la composition selon l'une quelconque des revendications 1 à 7 et un solvant (c'est-à-dire, une composition comprenant les constituants dans un état dissous plutôt qu'à l'état sec) sur une zone de test sur le support dans une première couche ;
(ii) élimination dudit solvant de la composition de la première couche ;
(iii) application d'une composition comprenant les constituants (c) à (e) de la composition selon l'une quelconque des revendications 1 à 7 et un solvant (c'est-à-dire, une composition comprenant les constituants dans un état dissous plutôt qu'à l'état sec) dans une deuxième couche sur la première couche ; et
(iv) élimination dudit solvant de la composition de la deuxième couche ; ou
(i) application d'une composition comprenant les constituants (a), (b), (d) et (e) de la composition selon l'une quelconque des revendications 1 à 7 et un solvant (c'est-à-dire, une composition comprenant les constituants dans un état dissous plutôt qu'à l'état sec) sur une zone de test sur le support dans une première couche ;
(ii) élimination dudit solvant de la composition de la première couche ;
(iii) application d'une composition comprenant les constituants (b) à (e) de la composition selon l'une quelconque des revendications 1 à 7 et un solvant (c'est-à-dire, une composition comprenant les constituants dans un état dissous plutôt qu'à l'état sec) dans une deuxième couche sur la première couche ; et
(iv) élimination dudit solvant de la composition de la deuxième couche ; ou
(i) application d'une composition comprenant les constituants (a), (d) et (e) de la composition selon l'une quelconque des revendications 1 à 7 et un solvant (c'est-à-dire, une composition comprenant les constituants dans un état dissous plutôt qu'à l'état sec) sur une zone de test sur le support dans une première couche ;
(ii) élimination dudit solvant de la composition de la première couche ;
(iii) application d'une composition comprenant les constituants (b) à (e) de la composition selon l'une quelconque des revendications 1 à 7 et un solvant (c'est-à-dire, une composition comprenant les constituants dans un état dissous plutôt qu'à l'état sec) dans une deuxième couche sur la première couche ; et
(iv) élimination dudit solvant de la composition de la deuxième couche ; ou
(i) application d'une composition comprenant les constituants (c) à (e) de la composition selon l'une quelconque des revendications 1 à 7 et un solvant (c'est-à-dire, une composition comprenant les constituants dans un état dissous plutôt qu'à l'état sec) sur une zone de test sur le support dans une première couche ;
(ii) élimination dudit solvant de la composition de la première couche ;
(iii) application d'une composition comprenant les constituants (a), (b), (d) et (e) de la composition selon l'une quelconque des revendications 1 à 7 et un solvant (c'est-à-dire, une composition comprenant les constituants dans un état dissous plutôt qu'à l'état sec) dans une deuxième couche sur la première couche ; et
(iv) élimination dudit solvant de la composition de la deuxième couche ; ou
(i) application d'une composition comprenant les constituants (b) à (e) de la composition selon l'une quelconque des revendications 1 à 7 et un solvant (c'est-à-dire, une composition comprenant les constituants dans un état dissous plutôt qu'à l'état sec) sur une zone de test sur le support dans une première couche ;
(ii) élimination dudit solvant de la composition de la première couche ;
(iii) application d'une composition comprenant les constituants (a), (b), (d) et (e) de la composition selon l'une quelconque des revendications 1 à 7 et un solvant (c'est-à-dire, une composition comprenant les constituants dans un état dissous plutôt qu'à l'état sec) dans une deuxième couche sur la première couche ; et
(iv) élimination dudit solvant de la composition de la deuxième couche ; ou
(i) application d'une composition comprenant les constituants (b) à (e) de la composition selon l'une quelconque des revendications 1 à 7 et un solvant (c'est-à-dire, une composition comprenant les constituants dans un état dissous plutôt qu'à l'état sec) sur une zone de test sur le support dans une première couche ;
(ii) élimination dudit solvant de la composition de la première couche ;
(iii) application d'une composition comprenant les constituants (a), (d) et (e) de la composition selon l'une quelconque des revendications 1 à 7 et un solvant (c'est-à-dire, une composition comprenant les constituants dans un état dissous plutôt qu'à l'état sec) dans une deuxième couche sur la première couche ; et
(iv) élimination dudit solvant de la composition de la deuxième couche.

12. Composition sèche selon l'une quelconque des revendications 1 à 7, élément de test diagnostique selon la revendication 8 ou 9 ou procédé selon la revendication 10 ou 11, l'acide pyridinecarboxylique réduisant une diminution de l'activité enzymatique d'au moins une enzyme dans la composition dans des conditions sèches et/ou humides.

13. Utilisation d'au moins un acide pyridinecarboxylique pour réduire une diminution de l'activité enzymatique d'au moins une enzyme dans une composition dans des conditions sèches et/ou humides, ladite composition comprenant une déshydrogénase, un cofacteur redox, un agent capable de provoquer un changement dans au moins une propriété mesurable d'un réactif indicateur en présence d'équivalents redox et un réactif indicateur, ledit acide pyridinecarboxylique étant un acide pyridinemonocarboxylique ou un acide pyridinedicarboxylique.

14. Utilisation selon la revendication 13, ledit au moins un acide pyridinecarboxylique dans la composition, dans des conditions sèches, étant compris dans un élément de test diagnostique, de préférence un élément de test diagnostique selon la revendication 8 ou 9.

15. Procédé pour déterminer la présence ou la quantité d'un analyte dans un échantillon de fluide corporel comprenant les étapes de :
(a) mise en contact de l'élément de test diagnostique selon la revendication 8 ou 9 avec un fluide corporel soupçonné de comprendre l'analyte dans des conditions appropriées pour transformer ladite au moins une enzyme en l'état reconstitué ;
(b) mesure d'un changement dans au moins une propriété mesurable du réactif indicateur dans la composition mouillée, comprenant ladite au moins une enzyme à l'état reconstitué sur l'élément de test diagnostique, la présence ou la quantité de l'analyte dans l'échantillon de fluide corporel étant déterminée.
